# EUROPEAN PATENT APPLICATION

(11) **EP 3 747 872 A1**
(43) Date of publication of application: **09.12.2020**
(21) Application number: 19178928.8
(22) Date of filing: 07.06.2019
(51) Int. Cl.: C07D 251/14, A61P 35/00, A61P 35/02

(54) **CARBOCYCLIC NUCLEOSIDE ANALOGUE**

(71) Applicant: Ludwig-Maximilians-Universität München, 80539 München (DE)
(72) Inventor: The designation of the inventor has not yet been filed
(74) Representative: Weiss, Wolfgang

(57) **Abstract**

The present invention relates to a novel hydrolytically stable carbocyclic 5-Aza-2-deoxycytidine as a hypomethylating agent.

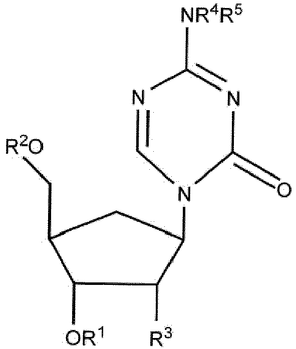

## Description

The present invention relates to novel hydrolytically stable carbocyclic nucleoside analogues of 5-aza-2'-deoxycytidine and 5-aza-cytidine and their use as hypomethylating agents.

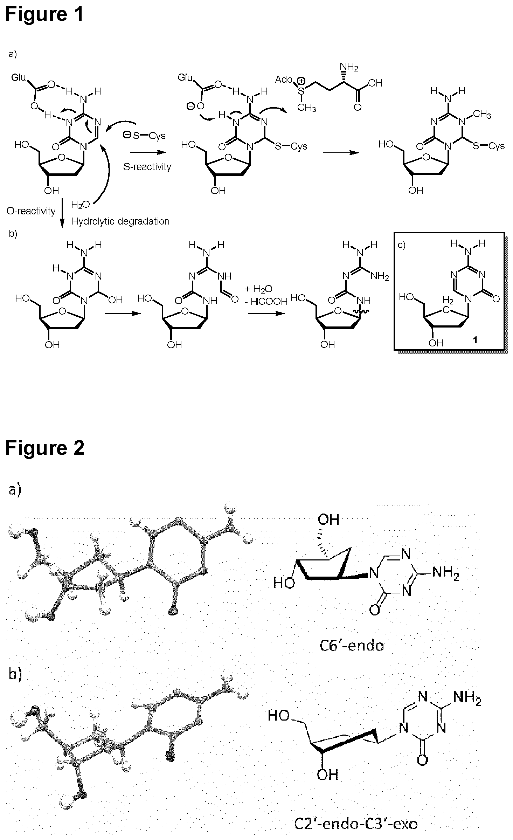

5-Aza-2'-deoxycytidine (decitabine, AzadC) and 5-aza-cytidine (AzaC) are nucleoside analogues that act as an antimetabolite by manipulating epigenetic information [1-5]. Epigenetic information in DNA is associated with the formation of 5-methyl-2'-deoxycytidine (mdC) from 2'-deoxycytidine (dC) with the help of DNA methyltransferases (DNMTs) and S-adenosylmethionine (SAM) as the methylating cofactor [6-7, 4]. Methylation of dC to mdC in promoter regions is typically associated with transcriptional silencing of genes [8-9]. AzadC is a prodrug that is inside cells converted into the corresponding active triphosphate and subsequently incorporated into the genome during cell division. AzaC is a prodrug as well, that is 2'-deoxygenated and subsequently converted into the corresponding 5'-triphosphate for incorporation into DNA. AzaC is also converted into the 5'-triphosphate without prior 2'-deoxygenation and incorporated into RNA. The mode of action of Aza(d)C involves reaction of its electrophilic C6 positions with a DNMT active site thiol nucleophile as shown in Fig. 1a [10-11]. This generates a covalent intermediate that is methylated by the SAM cofactor, hold in close proximity. Due to the N-atom at position 5 of the triazine heterocycle, the final beta-elimination reaction, which would usually release m(d)C from the DNMT enzyme is not possible anymore. The consequence is the formation of a covalent DNA-DNMT or RNA-DNMT crosslink.

As a result of administering Aza(d)C, a large drop of mdC levels, i.e. a hypomethylating effect is observed, which leads to the reactivation of silenced tumor suppressor genes in cancer cells [1], which allows re-differentiating cancer cells back into normally proliferating cells or gives rise to cell death. Aza(d)C is currently in use as a medicament for the treatment of myelodysplastic syndromes (MDS) [2] and acute myeloid leukemia (AML) [4]. Clinically, it is administered in several cycles, with each cycle involving one week of treatment and three weeks of pausing.

A problem associated with Aza(d)C is that it hydrolyses in aqueous solution following the path depicted in Fig. 1b. This hydrolysis compromises the activity of Aza(d)C, particularly over the long treatment times.

The present inventors have overcome this problem by providing analogues of Aza(d)C that can demethylate (and hence react with an S-nucleophile), while hydrolysis (reaction with an O-nucleophile) is blocked. Surprisingly, they have found that replacing the oxygen of the deoxyribose by a CH₂-group has a large remote effect on the reactivity of the heterocycle. These analogues, particularly a carbocyclic version of Aza(d)C still inhibit methylation by DNA methyltransferases but are hydrolytically stable as shown in Fig. 1c.

A first aspect of the present invention relates to a compound of Formula I: 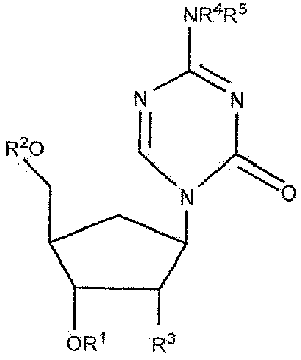
wherein R¹ is H, a phosphate group, or a hydroxyl-protecting group,
R² is H, a phosphate group, or a hydroxyl-protecting group,
R³ is H, F, CH₃, CH₂F, CHF₂, CF₃, OH or OR⁶, wherein R⁶ is a hydroxyl-protecting group, and
R⁴ and R⁵ are H or form an amino-protecting group,
or a salt thereof.

In the compounds of Formula I, R¹ may be selected from H, a phosphate group, e.g. a monophosphate, diphosphate or triphosphate group, or a hydroxyl-protecting group.

The term "phosphate group" includes an unmodified phosphate group, e.g. an unmodified monophosphate, unmodified diphosphate or unmodified triphosphate group or a modified phosphate group, e.g. a modified monophosphate, modified diphosphate or modified triphosphate group, wherein one or more oxygen atoms are replaced by carbon, sulfur and/or nitrogen atoms. Examples of modified phosphate groups are phosphonate, phosphoramidate or phosphorothioate groups. The term "phosphate group" also includes free phosphate groups and protected phosphate groups, e.g. phosphate esters including monoesters and diesters, and amidates including monoamidates, diamidates and amidate esters. Detailed reviews of protected phosphate and phosphonate groups are found in [28] and [29], the contents of which are herein incorporated by reference. Thus, in pharmaceutical applications, R¹ may be a group that is a protected phosphate, diphosphate or triphosphate group to liberate a phosphate, diphosphate or triphosphate group upon cleavage in the cell (prodrug concept).

Hydroxyl-protecting groups are known in the art, particularly in the field of nucleoside chemistry and include, but are not limited to, acyl groups such as acetyl or benzoyl, benzyl groups, trityl groups such as trityl, methoxytrityl or dimethoxytrityl, silyl groups such as trimethylsilyl, t-butyl-dimethylsilyl or tri-i-propysilyl, or alkyl- or substituted alkyl groups such as methyl, ethoxyethyl or beta-methoxyethoxymethyl.

R² may be selected from H, an unmodified or modified, free or protected phosphate group as described above, e.g. a monophosphate, diphosphate or triphosphate group, or a hydroxyl-protecting group as described above. Thus, in pharmaceutical applications, R² may be a group that is a protected phosphate diphosphate or triphosphate group to liberate a phosphate, diphosphate or triphosphate group upon cleavage in the cell (prodrug concept).

In certain embodiments, R³ may be H. In such a case, the 5-membered carbocyclic ring in Formula I can be considered as a deoxyribose wherein the oxygen atom at positon 1' of the ring has been replaced by a CH₂-group. In further embodiments, R³ may be OH or OR⁶, wherein R⁶ is a hydroxyl-protecting group as described above. In these embodiments, the 5-membered carbocyclic ring in Formula I may be considered as a ribose wherein the oxygen atom at positon 1' of the ring has been replaced by a CH₂-group. R³ may also be CH₃, CH₂F, CHF₂, CF₃ or F In these embodiments, the 5-membered carbocyclic ring in Formula I may be considered as a ribose analogue wherein the oxygen atom at positon 1' of the ring has been replaced by a CH₂-group.

R⁴ and R⁵ each may be H, thus providing a primary amino group NH₂ or they may form an amino-protecting group, e.g. wherein one or both H-atoms are replaced by the protecting group. Amino-protecting groups are known in the art, particularly in the field of nucleoside chemistry and include, but are not limited to, acyl groups such as acetyl or benzoyl, benzyl groups, carbobenzyloxy groups, tosyl groups, 9-fluorenylmethyloxycarbonyl groups or t-butyloxycarbonyl groups.

In certain embodiments, R¹ is H.

In certain embodiments, R² is H or an unmodified or modified phosphate group, e.g. a monophosphate, diphosphate or triphosphate group including a free or protected monophosphate, diphosphate or triphosphate group.

In certain embodiments, R³ is H or OH.

In certain particular embodiments R¹, R², R³, R⁴ and R⁵ each are H, or R¹, R², R⁴, and R⁵ each are H and R³ is OH. In an especially particular embodiment, R¹, R², R³, R⁴ and R⁵ are H. This compound is a carbocyclic analogue of AzadC (decitabine) which - as indicated above - has a surprisingly high hydrolytic stability while maintaining inhibitory activity towards methyltransferases, particularly DNA methyltransferases.

The present invention also encompasses salts of the compounds of Formula I, in particular pharmaceutically acceptable salts. The compounds of the present invention are capable of forming acid and/or base salts by means of the presence of amino and/or phosphate groups or groups similar thereto. Acid addition salts may be formed with inorganic acids and organic acids and include, but are not limited to, acetate, benzoate, hydrobromide, hydrochloride, citrate, lactate, phosphate, tosylate and trifluoroacetate salts. Base addition salts can be formed within inorganic bases and organic bases and include, but are not limited to, ammonium, sodium, potassium, calcium, magnesium salts or salts derived from primary, secondary and tertiary amines.

The compounds of the present invention act as antimetabolites by reducing the level of the epigenetic modification 5-methyl-2'-deoxycitidine (mdC). They may be incorporated into the genome and/or the RNA of proliferating cells and inhibit DNA- and/or RNA-methyltransferases leading to a reduction of mdC in DNA and mC in RNA. The loss of mdC in DNA, which is a transcriptional silencer in promoters, leads to the reactivation of genes including tumor suppressor genes, which elicits an anti-hyperproliferative effect.

Thus, a further aspect of the further invention relates to a compound of Formula I as described above or a pharmaceutically acceptable salt thereof for the use in medicine, e.g. in veterinary or human medicine.

In certain embodiments, the compound of the present invention is used for the treatment of a hyperproliferative disorder including malignant or non-malignant hyperproliferative disorders. The term *"hyperproliferative disorder"* relates to disorders caused by, associated with and/or accompanied by a dysfunctionally increased proliferation of cells, tissues and/or organs within an organism.

Thus, the invention also relates to a method of treating a hyperproliferative disorder, comprising administering to a subject in need thereof, particularly to a human subject, a therapeutically effective dose of a compound of Formula I or a pharmaceutically acceptable salt thereof.

In particular embodiments, the compounds of the present invention are used for the treatment of cancer. In certain embodiments, the compounds of the present invention are used for the treatment of leukemia, e.g. acute myeloid leukemia (AML) or a myelodysplastic syndrome (MDS) including previously treated or untreated, primary or in secondary MDS such as refractory anemia, refractory anemia with ringed sideroblasts, refractory anemia with excess blasts, refractory anemia with excess blasts in transformation and chronic myelomonocytic leukemia as well as intermediate-1, intermediate-2 and high risk MDS.

In further embodiments, the compounds of the invention may be used for the treatment of renal insufficiency based on results reported for decitabine [26]. In certain embodiments, the compounds of the present invention may be used for the treatment of atherosclerosis based on the observation that decitabine prevents atherosclerosis lesion formation and reduces the production of inflammatory cytokines by macrophages [27].

A compound of Formula I or a pharmaceutically acceptable salt thereof may be administered to a subject in need thereof as a pharmaceutically composition comprising the active agent and a pharmacologically acceptable carrier. The composition may be administered in any suitable way, e.g. orally, parenterally, by inhalation and/or by dermal application. The carrier may be any suitable pharmaceutical carrier. The compound of Formula I is administered in a therapeutically effective dose which may be determined by the skilled practitioner based on the subject and the disorder to be treated.

In pharmaceutical applications, the composition may be in the form of a solid dosage form, e.g. a tablet, a capsule etc. or a liquid dosage form, e.g. a solution, emulsion, suspension or the like.

A compound of Formula I may be administered in one or several treatment cycles, wherein each treatment cycle may involve at least 1, 2, 3, 4, 5, 6, 7 or 10 days.

A compound of Formula I may be administered as a monotherapy or in combination with a further medicament, particularly a medicament suitable for the treatment of hyperproliferative disorders as described above. In certain embodiments a compound of Formula I may be administered as a combination therapy together with an anti-cancer drug different from Formula I.

Further, the compounds of Formula I are also useful for non-medical applications, e.g. generally in the fields of basic research, diagnostics and/or drug screening. Thus, in certain embodiments, the invention relates to an in vitro use of a compound of Formula I for inhibiting the methylation of nucleic acids including DNA and RNA, particularly for inhibiting the methylation of DNA, more particularly for inhibiting the methylation of genomic DNA. Thus, the compounds are useful for a genome analysis in cells, e.g. eukaryotic cells such as animal cells including mammalian and human cells, particularly for an epigenetic analysis.

A general scheme for the preparation of the compounds of Formula I is depicted in the following scheme. The synthesis starts with a t-butyloxycarbonyl (Boc)-protected aminocyclopentane derivative 2 which has previously been used to synthesize DNA lesion analogues [12-15]. Compound 2 was first benzyl-protected to 3, Boc-deprotected to 4, and then reacted with carbimidazole 5, which was prepared in two steps from isomethylurea 6 after generation of the free base 7 with potassium hydroxide and reaction of 7 with carbonyldiimidazole. This provides the carbamoylurea-cyclopentane nucleoside analogue 8. Cyclization to the triazine base 9 was subsequently performed with triethylorthoformate. Reaction of 9 with NH₃ in methanol and deprotection of the benzyl groups with BCl₃ in dichloromethane furnished the final compound cAzadC 1 as the free nucleoside.

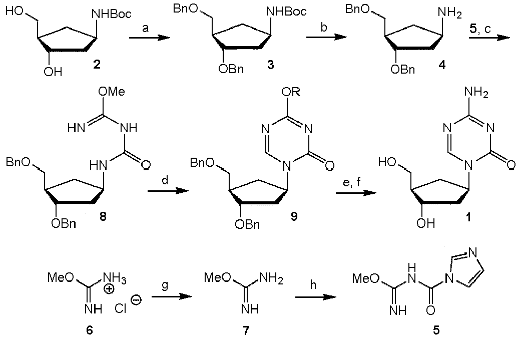

Recrystallization of compound cAzadC 1 from hot methanol gave colourless needles, which allowed us to solve the crystal structure that is depicted in Fig. 2. Interesting is the observation that cAzadC 1 exists with two different cyclopentane conformations in the crystal. One of these conformations is typical for 2'-deoxynucleosides in DNA showing that the cAzadC 1 nucleoside can adopt the correct DNA-type conformation and has the potential to get phosphorylated and integrated into the genome.

Further, the present invention shall be explained in more detailed by the following Figures and Examples.

### Figure legends

**Figure 1**. Depiction of 5-aza-2'-deoxycytidine (decitabine, AzadC) together with its mode of action. a) Active site thiol reacts with the C6-position of AzadC. b) Hydrolytic degradation pathway that goes in hand with reaction of a water molecule with the C6-position (O-reactivity) of AzadC. This leads to a final base loss and formation of an abasic site. c) Boxed, depiction of the carbocyclic version cAzadC 1.
**Figure 2**. Crystal structure of carbocyclic 5-aza-2'-deoxycytidine (cAzadC 1) showing the molecule in the observed C6'-endo conformation (a) and the C2'-endo conformation (2T1) (b).

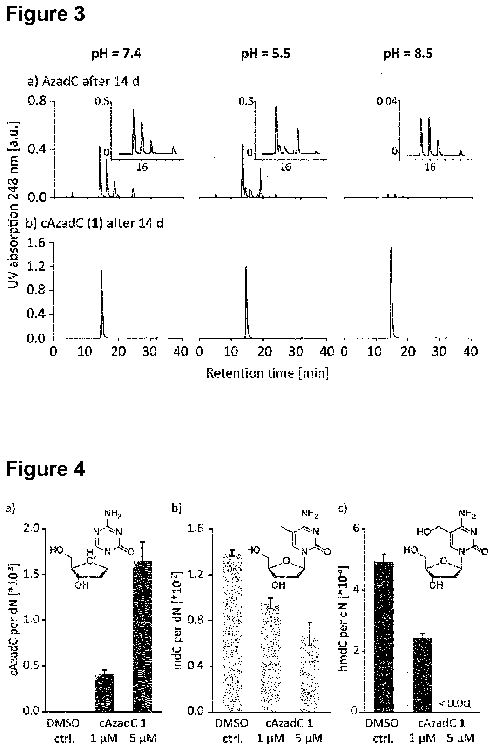

**Figure 3****.** HPLC-based stability measurements showing (a) severe hydrolytic decomposition of 5-aza-2'-deoxycytidine (AzadC) solutions at different pH values, while (b) the carbocyclic compound cAzadC 1 was stable at all three pH values. The inset table in (a) shows the chromatogram between t1 = 10 min and t2 = 20 min for AzadC. The AzadC signal is depicted in red.
**Figure 4****.** Depiction of the quantification data of DNA modifications of carbocyclic 5-aza-2'-deoxycytidine-treated (cAzadC 1) mouse embryonic stem cells (mESC) obtained by UHPLC-MS2. For each condition, three biological replicates were measured in technical triplicates. For each technical replicate, 0.5 µg of DNA were digested. Bar graphs represent mean, error bars represent standard deviation. LLOQ indicates the lower limit of quantification.

### Examples

### Example 1 - Synthetic Procedures

### Tert.-Butyl-N-[(1R,3S,4R)-3-hydroxy-4-(hydroxymethyl)cyclopentyl]-carbamate (2)

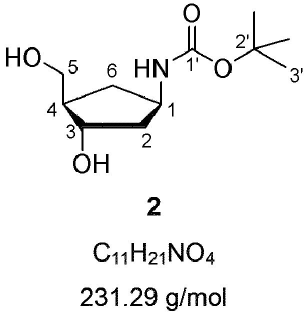

Compound **2** was synthesized from *tert*-Butyl-*N*-((1*S*,2*R*,4R,5*R*)-4-((*tert*-butyl-dimethylsilyloxy)methyl)-6-oxa-bicyclo-[3.1.0]-hex-2-yl)-carbamate as described previously [1].

### Tert.-Butyl-N-[(1R,3S,4R)-3-benzyloxy-4-(benzyloxymethyl)-cyclopentyl]-carbamate (3)

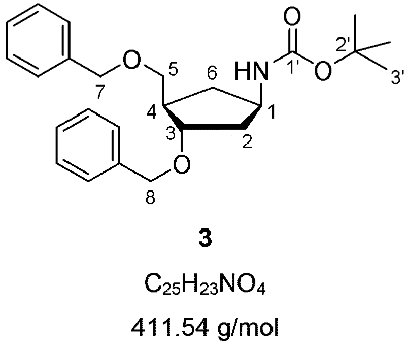

Boc-protected **2** (3.107 g, 13.43 mmol, 1.0 eq.) was dissolved in dry DMF (75 mL). The solution was cooled to 0 °C and NaH (60% dispersion in mineral oil, 1.182 g, 29.55 mmol, 2.2 eq.) was added in three portions. After 15 min at 0 °C, benzyl bromide (4.00 mL, 33.58 mmol, 2.5 eq.) was added dropwise. The reaction mixture was stirred for 1.5 h at 0 °C and for additional 2 h at rt. The reaction mixture was diluted using CH₂Cl₂ (30 mL) and the reaction was quenched with sat. NaHCO₃ (30 mL). The mixture was extracted with CH₂Cl₂ (500 mL). The organic phase was washed three times with sat. NaHCO₃ (1 × 400 mL, 2 × 100 mL) and dried over Na₂SO₄. The solvent was removed *in vacuo.* The crude product was purified by column chromatography on silica gel with a stepwise gradient *i*Hex/EtOAc (9:1→7:1→4:1) to afford product **3** as a colorless solid (3.39 g, 8.24 mmol, 61%).
**R_{f}** = 0.67 (iHex/EtOAc = 2:1)
**Mp** = 74 - 76 °C
**HRMS [ESI+]:** [C₂₅H₃₃NO₄Na]⁺, [M+Na⁺]⁺ calculated.: 434.2292, found: 434.2299
**¹H-NMR** (599 MHz, CDCl₃): *δ*/ppm = 7.39 - 7.26 (m, 10H, Ar-H), 4.76 (s, 1H, NH), 4.53 - 4.37 (m, 4H, C₇-H and C₈-H), 4.18 - 4.08 (m, 1H, C₁-H), 3.96 - 3.87 (m, 1H, C₃-H), 3.53 - 3.41 (m, 2H, C₅-H), 2.36 - 2.25 (m, 2H, C₆-Hₐ, C₄-H), 2.11 - 2.05 (m, 1H, C₂-Hₐ), 1.77 - 1.68 (m, 1H, C₂-H_{b}), 1.43 (s, 9H, C_{2'}-H), 1.29 - 1.20 (m, 1H, C₆-H_{b}).
**¹³C-NMR** (151 MHz, CDCl₃): *δ*/ppm = 155.5 (C_{1'}), 138.60, 138.23, 128.36, 128.28, 127.59, 127.56, 127.42 (C-Ar), 81.0 (C₃), 79.2 (C_{2'}), 73.3 (C₈), 71.9 (C₅), 71.3 (C₇), 50.4 (C₁), 44.9 (C₄), 39.6 (C₂), 35.0 (C₆), 28.6 (C_{2'}).
**FTIR (ATR):** *ṽ*/cm⁻¹ = 3339 (*w*), 2973 (*w*), 2929 (*w*), 2858 (*w*), 1692 (*m*), 1496 (*m*), 1453 (*m*), 1390 (*w*), 1364 (*m*), 1274 (*m*), 1247 (*m*), 1166 (*s*), 1091 (*s*), 1067 (*s*), 1027 (*m*), 1012 (*m*).

### (1R, 3S, 4R)-1-Amino-3-benzyloxy-4-(benzyloxymethyl)cyclopentane (4)

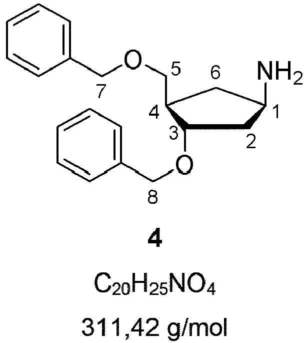

A solution of Boc-protected amine **3** (3.351 g, 8.14 mmol, 1.0 eq.) and trifluoroacetic acid (12 mL, 30% (v/v)) in dry CH₂Cl₂ (28 mL) was stirred for 1 h at rt. After removing the solvent *in vacuo,* the residue was resuspended in sat. Na₂CO₃ (100 mL) and stirred for 10 min. The mixture was extracted with EtOAc (3 × 150 mL) and the combined organic phases were dried over Na₂SO₄. Removing the solvent *in vacuo* resulted in the free amine **4** as viscous brown oil, which was used without further purification due to its instability towards O₂.

Analytical data of purified **4:**
**HRMS [ESI⁺]:** [C₂₃H₂₉N₄O₄Na]⁺, [M+Na⁺]⁺ calculated: 312.1958, found: 312.1955 **¹H-NMR** (400 MHz, CDCl₃): *δ*/ppm = 7.38 - 7.20 (m, 10H, C-Ar), 4.57 - 4.36 (m, 4H, C₇, C₈), 3.95 - 3.85 (m, 1H, C₁), 3.62 - 3.50 (m, 1H, C₃), 3.50 - 3.39 (m, 2H, C₅), 2.41 - 2.28 (m, 1H, C₄), 2.28 - 2.13 (m, 1H, C₆-Hₐ), 2.13 - 1.96 (m, 3H, NH₂, C₂-Hₐ), 1.65 - 1.50 (m, 1H, C₂-H_{b}), 1.19 - 1,.08 (m, 1H, C₆-Hₐ),
**¹³C-NMR** (101 MHz, CDCl₃): *δ*/ppm = 138.8, 138.4, 128.5, 128.5, 127.79, 127.75, 127.7, 127.6 (C-Ar), 81.82 (C₃), 73.23 (C₇), 72.58 (C₅), 71.10 (C₈), 51.40 (C₁), 45.63 (C₄), 42.07 (C₂), 38.08 (C₆).

### 2-Methyl-1-(1-imidazoylcarbonyl)-isourea (5)

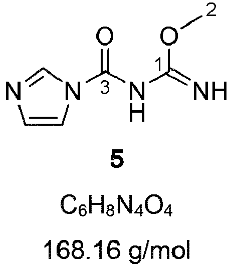

To a solution of O-methylisourea hydrochloride **6** (5.00 g, 45.65 mmol, 1.0 eq.) in Et₂O:H₂O (39:1) at - 15 °C, KOH powder (51.22 g, 912.96 mmol, 20.0 eq.) was added portion wise under constant stirring. After 30 min, the mixture was filtrated, and the residue was washed with ice-cold Et₂O (3 × 50 mL). The combined organic phases were concentrated to 20 mL *in vacuo.* Cooling to - 20 °C resulted in precipitation. Vacuum filtration under N₂ flow resulted in O-methylisourea **7** as a colorless wax (1.759 g, 23.74 mmol, 52%). A solution of **7** (1.24 g, 16.74 mmol, 1.0 eq.) and carbonyldiimidazole (2.90 g, 17.91 mmol, 1.07 eq.) in dry THF (35 mL) was stirred for 3 h at rt. After 1 h, a colorless precipitate was observed. The reaction mixture was concentrated to 3 mL *in vacuo* and filtrated. The residue was washed with ice-cold THF (2 × 3 mL) and the combined organic phases were lyophilized, which resulted in colorless solid **5** (1.946 g, 11.57 mmol, 69%).
**¹H-NMR** (400 MHz, CDCl₃): *δ*/ppm = 8.60 (br s, 1H, N-H), 8.35 (s, 1H, Ar-H), 7.58 (s, 1H, Ar-H), 7.04 (s, 1H, Ar-H), 6.04 (br s, 1H, N-H), 3.96 (s, 3H, C₂-H).
**¹³C-NMR** (101 MHz, CDCl₃): *δ*/ppm = 165.7 (C₁), 157.3 (C3), 137.5(Ar), 130.0 (Ar), 117.2 (Ar), 55.3 (C₂).

### 1-[(1'R,3'S,4'R)-3'-Benzyloxy-4'-(benzyloxymethyl)-cyclopentyl]-methylisobiuret (8)

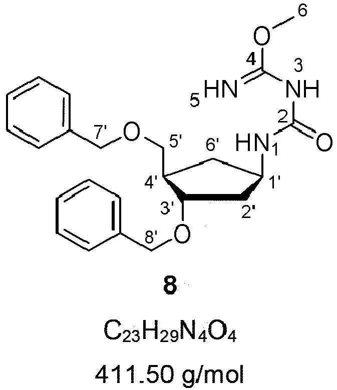

The crude product **4** was dissolved in dry acetonitrile (75 mL) and the carbonyl imidazole **5** (1.506 g, 8.96 mmol, 1.1 eq.) was added. The mixture was refluxed for 2 h. The solvent was removed *in vacuo.* The crude product was purified by column chromatography on silica gel with a stepwise gradient of *i*Hex/EtoAc (2:1→1:1) to obtain the methylisobiurete **8** as a yellow oil (2.613 g, 6.35 mmol, 78%).
**R_{f}** = 0.51 (DCM/MeOH = 19:1)
**HRMS [ESI⁺]**: [C₂₃H₂₉N₄O₄Na]⁺, [M+Na⁺]⁺ calculated: 434.2050, found: 434.2049
**¹H-NMR** (599 MHz, CDCl₃): *δ*/ppm = 7.35 - 7.16 (m, 10H, Ar-H), 5.42 (d,³*J*_{1',N1} = 7.8 Hz, 1H, N₁-H), 4.50 - 4.34 (m, 4H, C_{7'}-H_{a,b}, C_{8'}-H_{a,b}), 4.33 - 4.16 (m, 1H, C_{1'}-H), 3.93 - 3.81 (m, 1H, C_{3'}-H), 3.62 (s, 3H, C₆-H), 3.43 - 3.31 (m, 2H,C_{5'}-H), 2.34 - 2.18 (m, 2H, C_{6'}-Hₐ, C_{4'}-H), 2.06 (ddd, ²*J*_{2'a,2'b} = 13,0 Hz, ³*J*_{2'a,1'/3'} = 6,9 Hz, ³*J*_{2'a,1'/3'} = 4,6 Hz, 1H, C_{2'}-Hₐ), 1.73 (ddd, ²*J*_{2'a,2b'} = 13.0 Hz, ³*J*_{2'b,1'/3'} = 7.0 Hz, ³*J*_{2'b,1'/4'} = 7.0 Hz, 1H, C_{2'}-H_{b}), 1.28 - 1.11 (m, 1H, C_{6'}-H_{b}).
**¹³C-NMR** (151 MHz, CDCl₃): *δ*/ppm = 163.8 (C₂), 162.3 (C₄), 138.9 (C_{Ar}), 138.5 (C_{Ar}), 128.51 (C_{Ar}), 128.41 (C_{Ar}), 127.76 (C_{Ar}), 127.74 (C_{Ar}), 127.70 (C_{Ar}), 127.56 (C_{Ar}), 81.1 (C_{3'}), 73.3 (C_{7'}),71.9 (C_{5'}), 71.2 (C_{8'}), 53.8 (C₆), 49.5 (C_{1'}), 45.0 (C_{4'}), 39.5 (C_{2'}), 35.0 (C_{6'}).

### 4-Ethoxy-1-[(1'R,3'S,4'R)-3'-benzyloxy-4'-(benzyloxymethyl)-cyclopentyl]-1H-[1,3,5]-triazin-2-one (9a) and 4-methoxy-1-[(1'R,3'S,4'R)-3'-benzyloxy-4'-(benzyloxymethyl)-cyclopentyl]-1H-[1,3,5]-triazin-2-one (9b)

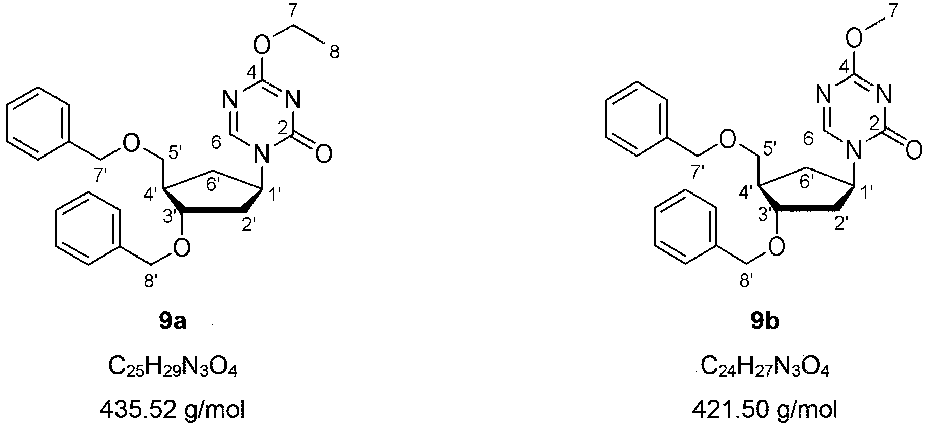

Trifluoroacetic acid (75 µL) was added to a solution of **8** (2.613 g, 6.35 mmol, 1.0 eq) in triethyl ortho-formiate (60 mL). The mixture was refluxed for 3 h. The solvent was removed *in vacuo.* The residue was co-evaporated once with MeOH and the crude product was purified by column chromatography on silica gel with a stepwise gradient of *i*Hex/EtOAc (2:1→1:1→1:2) to obtain **9a** (1.774 g, 4.07 mmol, 64%, colorless solid) and **9b** (268.0 mg, 0,64 mmol, 10%, colorless oil).
**9a**:
   **R_{f}** = 0.71 (iHex/EtOAc, 1:3)
   **HRMS [ESI⁺]:** [C₂₅H₃₀N₃O₄H]⁺, [M+H⁺]⁺ calculated: 436.2232, found.: 436.2231.
   **¹H-NMR** (400 MHz, CDCl₃): *δ*/ppm = 8.23 (s, 1H, C₆-H), 7.38 - 7.24 (m, 10H, Ar-H), 5.07 - 4.89 (m, 1H, C_{1'}-H), 4.56 - 4.39 (m, 6H, C_{7'}-H_{a,b}, C_{8'}-H_{a,b}, C₇-H_{a,b}), 4.07 - 3.08 (m, 1H, C_{3'}-H), 3.61 - 3.46 (m, 2H, C_{5'}-H_{a,b}), 2.52 - 2.36 (m, 2H, C_{6'}-Hₐ, C_{4'}-H), 2.28 (ddd, 1H, ²*J*_{2'a,2'b} = 13,3 Hz, ³*J* = 7,6 Hz, ³*J* = 2,7 Hz, C_{2'}-Hₐ), 2.11 (ddd, 1H, ²*J*_{2'a,2'b} = 13.3 Hz, ³*J* = 10.0 Hz, ³*J* = 6.3 Hz, C_{2'}-H_{b}), 1.84 - 1.68 (m, 1H, C_{6'}-H_{b}), 1.40 (t, 3H, ³*J*_{8,7} = 7,1 Hz, C₈-H),
   **¹³C-NMR** (101 MHz, CDCl₃): *δ*/ppm = 169.3 (C₄), 158.4 (C₆), 155.0 (C₂), 138.2 (C_{Ar}), 138.1 (C_{Ar}), 128.6 (C_{Ar}), 128.6 (C_{Ar}), 127.93 (C_{Ar}), 127.85 (C_{Ar}), 127.82 (C_{Ar}), 80.4 (C_{3'}), 73.4 (C_{7'/8'}), 71.26 (C_{7'/8'}), 71.25 (C_{5'}), 65.1 (C₇), 56.8 (C_{1'}), 44.9 (C_{4'}), 36.9 (C_{2'}), 32.6 (C_{6'}), 14.2 (C₈).
**9b**:
   **R_{f}** = 0.63 (iHex/EtOAc, 1:3)
   **HRMS [ESI⁺]:** [C₂₄H₂₈N₃O₄]⁺, [M+H⁺]⁺ calculated: 422.2074 , found: 422.2075.
   **¹H-NMR** (599 MHz, CDCl₃): *δ*/ppm = = 8.23 (s, 1H, C₆-H), 7.38 - 7.25 (m, 10H, Ar-H), 5.04 - 4.93 (m, 1H, C_{1'}-H), 4.57 - 4.43 (m, 6H, C_{7'}-H_{a,b}, C_{8'}-H_{a,b}), 4.09 - 3.98 (m, 4H, C_{3'}-H, C₇-H), 3.60 - 3.50 (m, 2H, C_{5'}-H_{a,b}), 2.50 - 2.39 (m, 2H, C_{6'}-Hₐ, C_{4'}-H), 2.28 (ddd, 1H, ²*J*_{2'a,2'b} = 13,1 Hz, ³*J* = 7,4 Hz, ³*J* = 2,7 Hz, C_{2'}-Hₐ), 2.11 (ddd, 1H, ²*J*_{2'a,2'b} = 13.1 Hz, ³*J* = 10.1 Hz, ³*J* = 6.3 Hz, C_{2'}-H_{b}), 1.80 - 1.68 (m, 1H, C_{6'}-H_{b}).
   **¹³C-NMR** (101 MHz, CDCl₃): *δ*/ppm = 169.9 (C₄), 158.5 (C₆), 155.0 (C₂), 138.3 (C_{Ar}), 138,0 (C_{Ar}), 128.63 (C_{Ar}), 128.57 (C_{Ar}), 127.93 (C_{Ar}), 127.84 (C_{Ar}), 127.82 (C_{Ar}), 80.4 (C_{3'}), 73.5 (C_{7'/8'}), 71.3 (C_{7'/8'}), 56.9 (C_{1'}), 56.0 (C₇), 44.9 (C_{4'}), 36.9 (C_{2'}), 32.6 (C_{6'}).

### 4-Amino-1-[(1'R,3'S,4'R)-3'-benzyloxy-4'-(benzyloxymethyl)-cyclopentyl]-1H-[1,3,5]triazin-2-one (10)

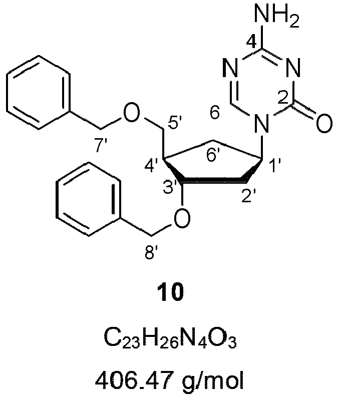

Ethoxytriazine **9a** (1.630 g, 3.74 mmol, 1.0 eq.) was dissolved in methanolic NH₃ (7 N in MeOH, 60 mL) and stirred for 3 h at rt. The mixture was diluted with H₂O (340 mL), resulting in a colorless precipitate, which was extracted wit EtOAc (3 × 200 mL). The organic phases were combined, dried and the solvent was removed *in vacuo,* which is resulted in the benzyl-protected cAzadC **10** (1.478 g, 3.63 mmol, 97%) as a colorless foam. Synthesis of **10** was also successful starting from the methoxytriazine **9b** and following the same procedure.
**R_{f}** = 0.50 (CH₂Cl₂/MeOH, 9:1)
**HRMS [ESI⁺]:** [C₂₃H₂₇N₄O₃]⁺, [M+H⁺]⁺ calculated: 407.2078, found.: 407.2081.
**¹H-NMR** (599 MHz, CDCl₃): *δ*/ppm = 8.01 (s, 1H, C₆-H), 7.35 - 7.23 (m, 10H, Ar-H), 5.78 (s, 2H, NH), 4.92 - 4.76 (m, 4H, C_{7'}-H and C_{8'}-H), 4.59 - 4.46 (m, 1H, C₁'-H), 4.07 - 3.95 (m, 1H, C_{3'}-H), 3.55 - 3.45 (m, 2H, C_{5'}-H), 2.44 - 2.28 (m, 2H, C_{6'}-Hₐ, C_{4'}-H), 2.24 - 2.16 (m, 1H, C_{2'}-Hₐ), 2.14 - 2.05 (m, 1H, C_{2'}-H_{b}), 1.78 - 1.67 (m, 1H, C_{6'}-H_{b}).
**¹³C-NMR** (151 MHz, CDCl₃): *δ*/ppm = 165.7 (C₄), 157.1 (C₆), 154.2 (C₂), 138.3, 138.3, 128.6, 128.5, 127.84, 127.78 (C-Ar), 80.4 (C_{3'}), 73.4 (C_{8'}), 71.4 (C_{5'}), 71.2 (C_{8'}), 56.7 (C_{1'}), 44.9 (C_{4'}), 36.8 (C_{2'}), 32.5 (C_{6'}).

### 4-Amino-1-[(1'R,3'S,4'R)-3'-hydroxy-4'-(hydroxymethyl)-cyclopentyl]-1H-[1,3,5]triazin-2-on (cAzadC, 1)

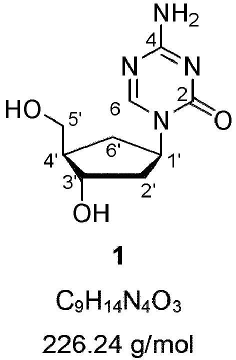

A solution of benzyl-protected **10** (1.478 g, 3.63 mmol, 1.0 eq) in CH₂Cl₂ (100 mL) was cooled to -78 °C and BCl₃ (1 M in DCM, 12.7 mL, 12.7 mmol, 3.5 eq.) was added dropwise. The mixture was stirred for 1 h at -78 °C and stirred for additional 2 h at rt. The reaction was quenched by addition of MeOH (85 mL) under constant stirring. The solvent was removed *in vacuo.* The crude product was purified by column chromatography on silica gel with a stepwise gradient CH₂Cl₂/MeOH (9:1→7:3) to obtain cAzadC (**1**, 0.740g, 3.27 mmol, 90%). Recrystallization from hot MeOH resulted in 57% cAzadC (**1**, 465.3 mg, 2.06 mmol) as colorless acicular monocrystalls.
**R_{f}** = 0.1 (CH₂Cl₂/MeOH, 9:1)
**Mp.:** 198-200 °C
**HRMS [ESI⁺]:** [C₉H₁₅N₄O₃]⁺, [M+H⁺]⁺ calculated: 227,1138, found.: 227,1139.
**¹H-NMR** (400 MHz, D₂O): *δ*/ppm = 8.29 (s, 1H, C₆-H), 4.90 - 4.62 (m, 1H, C_{1'}-H, Overlap with D₂O signal), 4.29 - 4.16 (m, 1H, C_{3'}-H), 3.73 (dd, ²*J*_{5'a,5'b} = 11.2 Hz, ³*J*_{5'a, 4'} = 5.7 Hz, 1H, C_{5'a}-H), 3.62 (dd, ²*J*_{5'a,5'b} = 11.2 Hz, ³*J*_{5'b, 4'} = 6.8 Hz, 1H, C_{5'b}-H), 2.41 - 2.29 (m, 1H, C_{6'a}-H), 2.28 - 2.18 (m, 1H, C_{2'a}-H), 2.17 - 2.02 (m, 2H, C_{2'b}-H, C_{4'b}-H), 1.77 - 1.55 (m, 1H, C_{6'b}-H).
**¹³C-NMR** (101 MHz, CDCl₃): *δ*/ppm = 165.5 (C₄), 158.5(C₆), 156.4(C₂), 72.2 (C_{3'}), 62.8 (C_{5'}), 56.2 (C_{1'}), 48.11 (C_{4'}), 38.0 (C_{2'}), 32.0 (C_{6'}).
**FTIR (ATR):** *ṽ*/cm⁻¹ = 3194 *(m),* 1653 *(m),* 1540 (s), 1437 (s), 1278 (s), 1036 (s).

### Example 2

### X-ray crystal structure analysis of cAzadC

The cAzadC monocrystals were analysed by X-ray crystallography ( **Table** 1). The solved structure shows the cAzadC 1 in two different cyclopentane conformations in the crystal. One conformer adopts a C6'-endo (P = 88.2 °, max = 47.8 °) conformation (Fig. 2a), while the second exists as the C2'-endo-C3'-exo (South, P = 150.8 °, max = 45.4 °) conformer (Fig. 2b). The latter conformation is typical for 2'-deoxynucleosides in DNA.

Further figures were generated with the program *Mercury 3.5.1.* (*Cambridge Crystallographic Data Center*). The data are deposited under CCDC 1910952.

**Table 1: Crystallographic data for cAzadC**

| net formula | C₉H₁₄N₄O₃ | transmission factor range | 0.9087-0.9580 |
|---|---|---|---|
| *M*ᵣ/g mol⁻¹ | 226.24 | refls. measured | 34579 |
| crystal size/mm | 0.100 × 0.080 × 0.030 | *R*ᵢₙₜ | 0.0373 |
| *T*/K | 100(2) | mean σ(*I*)/*I* | 0.0197 |
| radiation | MoKα | θ range | 3.031-25.40 |
| diffractometer | 'Bruker D8Venture' | observed refls. | 3474 |
| crystal system | orthorhombic | *x, y* (weighting scheme) | 0.0372, 0.4868 |
| space group | 'P 21 21 21' | hydrogen refinement | mixed |
| *a*/Å | 8.4451(3) | Flack parameter | 0.3(3) |
| *b*/Å | 11.0958(4) | refls in refinement | 3787 |
| *c*/Å | 21.9635(8) | parameters | 321 |
| α/° | 90 | restraints | 0 |
| β/° | 90 | *R*(*F*_{obs}) | 0.0284 |
| γ/° | 90 | *R*_{w}(*F*²) | 0.0699 |
| *V*/Å³ | 2058.09(13) | *S* | 1.054 |
| *Z* | 8 | shift/errorₘₐₓ | 0.001 |
| calc. density/g cm⁻³ | 1.460 | max electron density/e0.180 Å⁻³ | |
| µ/mm⁻¹ | 0.112 | min electron density/e -0.173 Å⁻³ | |

absorption correction multi-scan

### Example 3 - Test for hydrolytic stability

We next investigated the hydrolytic stability of cAzadC 1 in direct comparison to the pharmaceutical AzadC.

Both nucleosides were dissolved in aqueous KH₂PO₄ buffer (100 mM, pH = 7.4, 5.5 and 8.5) in 100 mM concentration. The solutions were immediately analysed *via* HPLC (t=0 h) with subsequent HPLC analyses every 1.5 h or at indicated time points. As stationary phase an EC 250/4 NUCLEOSIL 120-3 C18 (*Macherey-Nagel*) chromatography column was used. The mobile phase consisted of water (buffer A) and acetonitrile (buffer B) at a flow-rate of 0.5 mL/min as follows: 0→25 min, 0→5% buffer B; 25 min→28 min, 5%→80%; 28 min→38 min, 80%; 38 min→43 min 80%→0%; 43 min→45 min, 0%.

Since one treatment cycle for AzadC goes over four weeks we decided to measure the stability at a time point related to a half cycle (14 d). NMR spectra were measured after keeping the solutions at r.t.

As tumour cells often provide an acidic micro-environment [16], the stability under slightly acidic pH is particularly informative. As evident from the data shown in Fig. 3, the pharmaceutical AzadC strongly degraded within these 14 d. Importantly, at pH = 5.5 and at pH = 8.5, intact AzadC was only hardly detectable anymore. At physiological pH (7.4), AzadC was still present after 14 d but the level of degradation is dramatic.

In contrast to these results, we observed for cAzadC **1** surprisingly no degradation at all tested pH values, including pH = 5.5. This result led to the surprising discovery that the simply O → CH₂ exchange causes a strong remote disarming effect that seems to change the properties of the triazine ring so that reaction with water is stopped.

### Example 4 - Test for biological functions

### Cell culture of mESC for cAzadC treatment

Feeder independent wt J1 (strain 129S4/SvJae) [24] cells were cultured in the presence of serum and LIF as previously described [25]. They were routinely maintained on gelatinized plates in 2i/L medium. For priming experiments, 2i cultures were passaged when applicable in DMEM supplemented with FBS and LIF as above but lacking the inhibitors. For drug treatment, cells were moved into the primed state by removing 2i from the medium. Cells were incubated 2 d in DMEM supplemented with FBS and LIF in 6-well plates (*VWR*). After splitting, 2x10⁵ cells were transferred into a 6-well plate culture dish and supplemented with either 1 µM (in 0.01% DMSO) or 5 µM cAzadC (in 0.05% DMSO) and treated for 72 h. After removal of the medium and washing the cells with DPBS, they were directly lysed with RLT⁺ buffer as described in a previous publication [18].

### gDNA isolation, total enzymatic digest and UHPLC-MS²

The gDNA was isolated as described previously [18]. The gDNA was directly subjected to a total enzymatic digest and analyzed using UHPLC-MS² as described in a previous publication [19]. The external calibration curve was generated by serially diluting pure cAzadC [20 pmol, 10 pmol, 5 pmol, 2.5 pmol, 1.25 pmol, 0.625 pmol, 0.3125 pmol] and measuring it in technical triplicates prior to each measurement. Linear regression was done by *OriginPro 2016G.*

### Results

We investigated if the disarming effect described in Example 3 would influence the biological functions. We used for this purpose mouse embryonic stem cells (mESC) that were primed in serum/Lif as a model system, since mdC levels increase from naïve to primed state [17]. We added cAzadC 1 in two different concentrations (1 µM and 5 µM) to mESC that have been primed for 48 h and allowed the cells to further proliferate under priming conditions in the presence of cAzadC 1 for additional 72 h. After the 72 h, we harvested the cells, isolated the DNA and digested the DNA down to the nucleoside level using our described protocol [18].

The levels of mdC were finally precisely quantified using isotope dilution UHPLC-MS². To this end, isotopically labelled standards of the nucleosides were spiked-in for exact quantification [19, 18]. In addition to mdC, we quantified the levels of 5-hydroxymethyl-2'-deoxycytidine (hmdC), which is formed from mdC by the action of TET enzymes [20-21]. The absolute levels of hmdC are in mESC more than ten times lower than the mdC levels [20, 22]. The consequence is that even after a substantial reduction of mdC, there should be sufficient mdC to keep the hmdC levels constant. The question if and by how much the hmdC level is affected can therefore inform us about how epigenetic reprogramming is organized.

Parallel to the quantification of mdC and hmdC we also quantified to which extend cAzadC 1 itself was incorporated into the genome of the mESC. Detection of AzadC in the genome of treated cells is only possible after treatment of the DNA with NaBH₄. Application of NaBH₄ reduces the C(5)=C(6) double bond, which stabilizes the compound so that its quantification becomes possible [23, 19]. To our delight, we noted that the stability of cAzadC 1 allowed its quantification without this pre-treatment. We also noted that the applied enzymatic digestion protocol allowed to digest genomic DNA (gDNA) even in the presence of large amounts of cAzadC 1. Taken together, quantification of cAzadC 1 by UHPLC-MS² using an external calibration curve was possible in parallel to quantification of canonical and epigenetic bases.

At 1 µM cAzadC 1 concentration, we detected a cAzadC 1 level of 5x10⁻⁴ cAzadC per dN (Fig. 4a). This amounts to almost 3 million cAzadC nucleotides integrated into the genome. At the higher concentration of 5 µM cAzadC 1, the level increased 3-fold to 1.7x10⁻³ cAzadC per dN and consequently to more than 8 million integrated cAzadCs per genome. Compared to the incorporation of AzadC, which reaches 1.2x10⁻³ AzadC per dN, when applied with 1 µM [19], the levels of cAzadC 1 reaches about a third of this level. The data clearly show that the carbocyclic version of AzadC (cAzadC 1) is incorporated and that it reaches in the genome finally comparable levels at 5 µM concentration.

Importantly, after exposing the mESC for 72 h at 1 M cAzadC in the medium, we detected a reduction of the mdC values by almost 30% (Fig. 4b). At 5 M concentration in the medium, the mdC levels dropped even to about 50% of the original value. A decrease to 50% is observed for AzadC as well. Here, however, the 50%-reduction is reached faster (24 h) and already with lower AzadC concentration (1 µM) [19].

The data show that the carbocyclic version cAzadC 1 needs simply more time to affect the mdC levels by the same amount. We believe that this effect is caused by a potentially slower conversion of cAzadC 1 into the triphosphate. The slower kinetics of cAzadC 1, however, is not necessarily a disadvantage given the long treatment times that are applied in the clinic.

Very interesting is also the discovery that the hmdC levels were reduced to about 50% already in the 1 µM experiment. At 5 µM, we were even unable to detect hmdC above background levels using 0.5 µg of genomic DNA. The result shows that the hmdC level dropped even faster than the mdC levels, although hmdC is more than ten times less abundant in the genome. This result is interesting. It indicates that hmdC might be potentially predominantly generated in the mdC maintenance process during cell division. We see here that compound cAzadC 1 is a perfect tool molecule that now allows to gain further insight into the interplay between methylation of dC to mdC and oxidation of mdC to hmdC. With the new compound cAzadC 1 in hand we can cleanly correlate demethylation of the genome with the corresponding cellular effects without compromising DNA damaging effects. Finally, cAzadC 1 may not only be a valuable tool compound but even a next generation epigenetic pharmaceutical.

### Summary

We show that replacement of the in-ring O-atom by a CH₂-unit stabilizes the pharmaceutical so that its nucleophilic reaction with water is stopped. The new nucleoside cAzadC 1 is accepted by the phosphorylating enzymes in cells and the corresponding cAzadC-triphosphates are efficiently incorporated into the genome. cAzadC 1 is incorporated in the genome with several million nucleotides and it causes the mdC level to decrease to 70% relative to the control levels.

### List of references

[1] M. Daskalakis, T. T. Nguyen, C. Nguyen, P. Guldberg, G. Kohler, P. Wijermans, P. A. Jones, M. Lubbert, Blood 2002, 100, 2957-2964.
[2] H. Kantarjian, J. P. Issa, C. S. Rosenfeld, J. M. Bennett, M. Albitar, J. DiPersio, V. Klimek, J. Slack, C. de Castro, F. Ravandi, R. Helmer, 3rd, L. Shen, S. D. Nimer, R. Leavitt, A. Raza, H. Saba, Cancer 2006, 106, 1794-1803.
[3] C. Stresemann, F. Lyko, Int. J. Cancer 2008, 123, 8-13.
[4] Y. Koh, Y. A. Kim, K. Kim, J.-A. Sim, S.-S. Yoon, S. M. Park, Y. H. Yun, Blood 2016, 128, 2381-2381.
[5] M. Nieto, P. Demolis, E. Béhanzin, A. Moreau, I. Hudson, B. Flores, H. Stemplewski, T. Salmonson, C. Gisselbrecht, D. Bowen, F. Pignatti, Oncologist 2016, 21, 692-700.
[6] S. S. Smith, B. E. Kaplan, L. C. Sowers, E. M. Newman, Proc. Natl. Acad. Sci. 1992, 89, 4744-4748.
[7] G. G. Wilson, R. J. Roberts, S. Kumar, J. Posfai, M. Sha, S. Klimasauskas, X. Cheng, Nucleic Acids Res. 1994, 22, 1-10.
[8] A. Bird, Genes Dev. 2002, 16, 6-21.
[9] R. J. Klose, A. P. Bird, Trends Biochem. Sci. 2006, 31, 89-97.
[10] R. Jüttermann, E. Li, R. Jaenisch, Proc. Natl. Acad. Sci. 1994, 91, 11797-11801.
[11] S. Gabbara, A. S. Bhagwat, Biochem. J. 1995, 307, 87-92.
[12] M. Ober, H. Müller, C. Pieck, J. Gierlich, T. Carell, J. Am. Chem. Soc. 2005, 127, 18143-18149.
[13] H. Müller, T. Carell, Eur. J. Org. Chem. 2007, 2007, 1438-1445.
[14] F. Büsch, J. C. Pieck, M. Ober, J. Gierlich, G. W. Hsu, L. S. Beese, T. Carell, Chem. Eur. J. 2008, 14, 2125-2132.
[15] T. H. Gehrke, U. Lischke, K. L. Gasteiger, S. Schneider, S. Arnold, H. C. Müller, D. S. Stephenson, H. Zipse, T. Carell, Nat. Chem. Bio. 2013, 9, 455.
[16] Y. Kato, S. Ozawa, C. Miyamoto, Y. Maehata, A. Suzuki, T. Maeda, Y. Baba, Cancer Cell Int. 2013, 13, 89.
[17] S. Takahashi, S. Kobayashi, I. Hiratani, Cell. Mol. Life Sci. 2018, 75, 1191-1203.
[18] F. R. Traube, S. Schiffers, K. Iwan, S. Kellner, F. Spada, M. Müller, T. Carell, Nat. Protoc. 2019, 14, 283-312.
[19] S. Schiffers, T. M. Wildenhof, K. Iwan, M. Stadlmeier, M. Müller, T. Carell, Helv. Chim. Acta 2019, 102, e1800229.
[20] M. Tahiliani, K. P. Koh, Y. Shen, W. A. Pastor, H. Bandukwala, Y. Brudno, S. Agarwal, L. M. Iyer, D. R. Liu, L. Aravind, A. Rao, Science 2009, 324, 930-935.
[21] S. Ito, L. Shen, Q. Dai, S. C. Wu, L. B. Collins, J. A. Swenberg, C. He, Y. Zhang, Science 2011, 333, 1300-1303.
[22] M. Münzel, D. Globisch, T. Carell, Angew. Chem. Int. Ed. Engl. 2011, 50, 6460-6468.
[23] A. Unnikrishnan, A. N. Q. Vo, R. Pickford, M. J. Raftery, A. C. Nunez, A. Verma, L. B. Hesson, J. E. Pimanda, Leukemia 2018, 32, 900-910.
[24] E. Li, T. H. Bestor, R. Jaenisch, Cell 1992, 69, 915-926.
[25] T. Pfaffeneder, B. Hackner, M. Truss, M. Münzel, M. Müller, C. A. Deiml, C. Hagemeier, T. Carell, Angew. Chem. Int. Ed. Engl. 2011, 50, 7008-7012.
[26] Ravandi, F.; Kantarjian, J. E.; Issa, S.; Jabbour, S.; Santos, G.; McCue, D.; Garcia-Manero, F. P. S.; Pierce, E.; O'Brien, J. P.; Cortés, J. E.; Ravandi, F. (2010). "Feasibility of Therapy with Hypomethylating Agents in Patients with Renal Insufficiency". Clinical Lymphoma, Myeloma & Leukemia. 10 (3): 205-210.
[27] Dunn, J; Thabet, S; Jo, H (July 2015). "Flow-Dependent Epigenetic DNA Methylation in Endothelial Gene Expression and Atherosclerosis". Arteriosclerosis, Thrombosis, and Vascular Biology. 35 (7): 1562-9.
[28] Hecker, S.J.; Erion, M.D. (2008), "Prodrugs of phosphates and phosphonates". J. Med. Chem 51: 2328-2345.
[29] Wiemer, J.; Wiemer D.F. (2015), "Prodrugs of phosphonates and phosphates: Crossing the membrane barrier". Top. Curr. Chem. 360: 115-160.

## Claims

1. A compound of Formula I 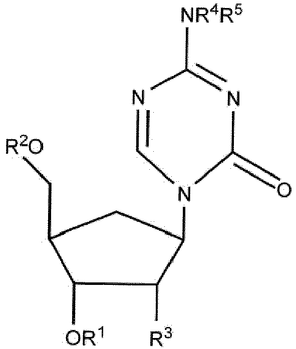
wherein R¹ is H, a phosphate group or a hydroxyl-protecting group,
R² is H, a phosphate group, or a hydroxyl-protecting group,
R³ is H, F, CH₃, CH₂F, CHF₂, CF₃, OH or OR⁶ wherein R⁶ is a hydroxyl-protecting group,
R⁴ and R⁵ each are H or form an amino-protecting group,
or a salt thereof.

2. The compound of claim 1 wherein each phosphate group is independently selected from:
(i) a free or protected unmodified phosphate, e.g. monophosphate, diphosphate or triphosphate group, and
(ii) a free or protected modified phosphate, e.g. monophosphate, diphosphate or triphosphate group, wherein the modified phosphate group may be selected from a phosphonate, phosphoramidate or phosphorothioate group.

3. The compound of claim 1 or 2 wherein R¹ is H.

4. The compound of any one of claims 1-3 wherein R² is H.

5. The compound of any one of claims 1-4 wherein R³ is H or OH.

6. The compound of any one of claims 1-5 wherein R⁴ and R⁵ are H.

7. The compound of any one of claims 1-6 wherein R¹, R², R³, R⁴ and R⁵ each are H, or wherein R¹, R², R⁴ and R⁵ each are H and R³ is OH.

8. The compound of any one of claims 1-7 wherein R¹, R², R³, R⁴ and R⁵ each are H.

9. The compound of any one of claims 1-8 or a pharmaceutically acceptable salt thereof for use in medicine.

10. The compound of any one of claims 1-8 or a pharmaceutically acceptable salt thereof for the treatment of a hyperproliferative disorder.

11. The compound of any one of claims 1-8 or a pharmaceutically acceptable salt thereof for the treatment of cancer.

12. The compound of any one of claims 1-8 or a pharmaceutically acceptable salt thereof for the treatment of acute myeloid leukemia (AML), or a myelodysplastic syndrome (MDS).

13. The compound of any one of claims 1-8 or a pharmaceutically acceptable salt thereof for the treatment of atherosclerosis or renal insufficiency.

14. An in vitro use of the compound of any one of claim 1-8 for inhibiting the methylation of nucleic acids, particularly of DNA.

15. The use of claim 11 for an epigenetic analysis.
